# DEMANDE DE BREVET EUROPEEN

(11) **EP 2 601 889 A1**
(43) Date de publication de la demande: **12.06.2013**
(21) Numéro de dépôt: 12193722.1
(22) Date de dépôt: 22.11.2012
(51) Int. Cl.: A61B 5/12, G06F 3/033, G06F 3/01

(54) **Dispositif, système et procédé de notation en temps réel d'un stimulus**

(30) Priorité: 08.12.2011 FR 1161360
(71) Demandeur: PEUGEOT CITROËN AUTOMOBILES SA, 78140 Velizy-Villacoublay (FR)
(72) Inventeur: Renard, Cyril, 92100 BOULOGNE BILLANCOURT (FR); Lafon, Brice, 92500 RUEIL MALMAISON (FR)

(57) **Abrégé**

L'invention concerne un dispositif de notation (3) en temps réel d'un stimulus, comportant un bouton monté rotatif sur un boîtier. Le boîtier présente une échelle graduée associée au mouvement du bouton. L'invention se rapporte également à un système de notation en temps réel d'un stimulus, notamment d'un stimulus auditif, de préférence encore d'un bruit de combustion d'un moteur Diesel d'un véhicule automobile, comprenant des moyens d'émission (1) vers un utilisateur (2) du stimulus à évaluer, un dispositif de notation en temps réel et des moyens de synchronisation (4) pour synchroniser le stimulus et les notations de l'utilisateur. L'invention vise encore un procédé de notation en temps réel d'un stimulus mis en oeuvre au moyen d'un tel système de notation.

## Description

La présente invention concerne un dispositif, un système et un procédé de notation en temps réel d'un stimulus, notamment d'un stimulus auditif.

Dans le domaine automobile, par exemple, le bruit de combustion inhérent aux motorisations Diesel est aujourd'hui à l'origine de plaintes de clients.

Il est connu des logiciels permettant de simuler le bruit de combustion en associant une note à un profil de pression de cylindre pour un cas de fonctionnement et une calibration donnés. Cette note peut être estimée grâce à l'apprentissage d'un modèle consistant à coter subjectivement, différents sons correspondants à des signaux de pression cylindre pour différents points de fonctionnement dans le plan régime/couple.

Ces logiciels sont adaptés pour simuler des points de fonctionnement stabilisés à chaud ou à froid du moteur, permettant ainsi de minimiser les bruits de combustion en fonctionnement stabilisé. Malgré cela, le bruit de combustion est encore aujourd'hui très souvent critiqué pendant les phases de fonctionnements transitoires durant lesquelles la qualité du bruit de combustion peut varier de manière continue ou discontinue dans le temps.

Il est possible d'adapter les logiciels aux cas de fonctionnements transitoires en les implémentant avec des données subjectives relatives à la gêne occasionnée par les bruits de fonctionnement du moteur en phase transitoire.

L'évaluation subjective d'un stimulus continu est traditionnellement réalisée par une cotation jury après présentation du stimulus. Il en résulte une cotation unique et globale pour tout le stimulus qui ne représente pas l'évolution temporelle de celui-ci. Les moyens d'évaluations actuels ne permettent donc pas d'évaluer les bruits de combustion en fonctionnement transitoire.

Par ailleurs, il est connu des logiciels (par exemple les logiciels « Shinji » ou « ExpSuite ») d'acquisition en temps réel des paramètres psychoacoustiques sur un échantillon. Le logiciel Expsuite, notamment, permet aux utilisateurs de noter les stimuli auditifs au moyen d'un ordinateur et d'une interface avec l'ordinateur, laquelle peut prendre la forme d'un clavier, d'une souris ou d'une manette de jeux (« joystick »).

Cependant, ces logiciels nécessitent que l'utilisateur soit présent devant l'ordinateur pour noter les différents stimuli auditifs. Ces dispositifs présentent ainsi un encombrement important. En outre, leur utilisation est reste complexe.

Le but de la présente invention est de fournir un dispositif, un système et un procédé permettant d'éviter les inconvénients précités.

Plus particulièrement, l'invention concerne un dispositif, un système et un procédé permettant de noter en temps réel un stimulus, notamment un stimulus auditif, sans que l'utilisateur ait besoin d'utiliser l'écran d'un ordinateur.

A cette fin, la présente invention propose un dispositif de notation en temps réel d'un stimulus, remarquable en ce qu'il comporte un bouton apte à être déplacé par rapport à un boîtier, le boîtier présentant une échelle graduée associée au déplacement du bouton.

Selon une alternative, l'un parmi le bouton et le boîtier présente des crans aptes à coopérer avec une piste crantée de l'autre parmi le bouton et le boîtier, l'écart angulaire entre deux crans correspondant de préférence à une division d'une graduation de l'échelle graduée.

Selon une alternative, le dispositif comprend un dispositif de retour d'effort agissant sur le bouton.

Selon une variante, le dispositif de retour d'effort est du type variable, le retour d'effort étant fonction du, de préférence proportionnel au, déplacement du bouton.

Selon une variante, le dispositif de notation comprend des moyens de retour du bouton en une position neutre du bouton.

Selon une variante, le dispositif de retour d'effort et les moyens de retour du bouton en une position neutre du bouton sont formés de deux ressorts de rappels montés entre le boîtier et le bouton.

Selon une variante, le dispositif de notation comporte des moyens de codage du déplacement du bouton et des moyens de transmission électroniques du déplacement codé vers une unité de traitement.

Selon une variante, le bouton est monté rotatif par rapport au boîtier.

Selon un autre aspect, l'invention propose un système de notation en temps réel d'un stimulus, notamment d'un stimulus auditif, de préférence encore d'un bruit de combustion d'un moteur Diesel d'un véhicule automobile, comprenant :
- des moyens d'émission vers un utilisateur du stimulus à évaluer ;
- un dispositif de notation en temps réel du stimulus, tel que décrit ci-avant dans toutes ses combinaisons ; et
- des moyens de synchronisation pour synchroniser le stimulus et les notations de l'utilisateur.

Selon une variante, les moyens de synchronisation comportent un ordinateur agencé pour :
- commander la diffusion du stimulus ; et
- recevoir les notations de l'utilisateur en temps réel.

L'invention se rapporte encore à un procédé de notation en temps réel d'un stimulus, notamment d'un stimulus auditif, de préférence d'un bruit de combustion d'un moteur Diesel d'un véhicule automobile, mis en oeuvre au moyen d'un système de notation tel que décrit ci-dessus dans toutes ses variantes, comprenant les étapes de :
- émission d'un stimulus vers un utilisateur ;
- notation par l'utilisateur en temps réel du stimulus ;
- synchronisation du stimulus et de la notation du stimulus de sorte à obtenir une corrélation entre au moins une portion du stimulus et une notation associée.

D'autres caractéristiques et avantages de l'invention apparaîtront à la lecture de la description qui suit d'un mode de réalisation préféré de l'invention, donnée à titre d'exemple et en référence aux figures annexés qui représentent :
- la figure 1, un schéma d'un système de notation en temps réel ;
- les figures 2 et 3, des schémas d'un exemple de dispositif de notation en temps réel pouvant être mis en oeuvre dans le système de la figure 1.

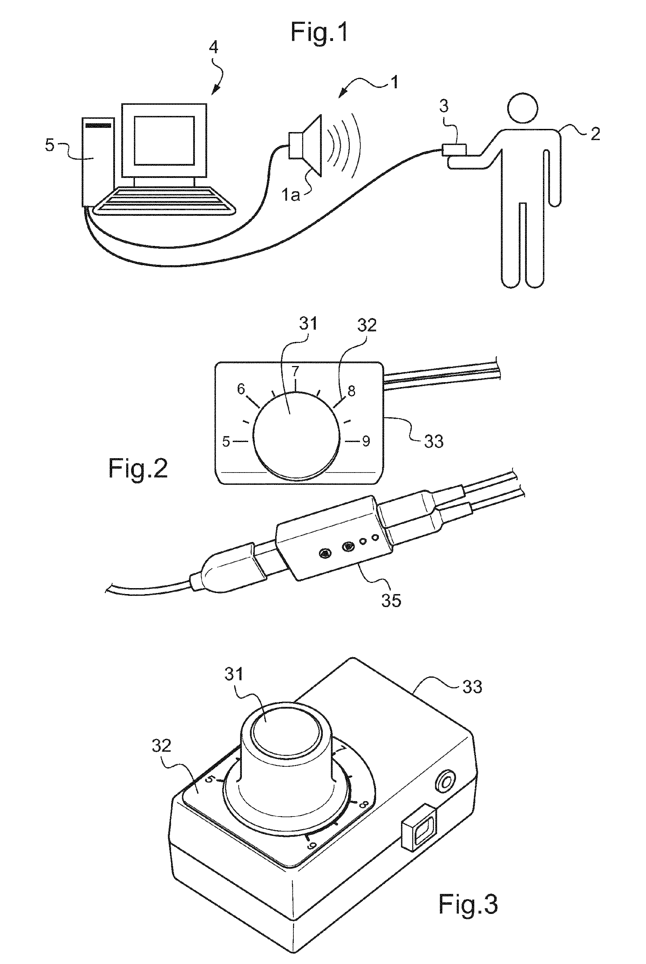

La figure 1 illustre schématiquement un système de notation en temps réel d'un stimulus auditif. Le système de notation en temps réel comprend des moyens d'émission 1 d'un stimulus auditif à évaluer vers un utilisateur 2, ici un haut parleur 1a. Le système de notation en temps réel comprend en outre un dispositif de notation en temps réel 3 du stimulus auditif pour que l'utilisateur 2 puisse noter en temps réel les différents bruits successifs composant le stimulus auditif. En d'autres termes, l'utilisateur 2 peut modifier la note attribuée au stimulus durant celui-ci, notamment si celui présente des variations de niveaux sonores. Cette modification de la note attribuée peut intervenir à tout instant pendant le stimulus. La note attribuée au stimulus peut ainsi être variable avec le temps, c'est-à-dire sur la durée du stimulus. Le système de notation comprend également des moyens de synchronisation 4 pour synchroniser le stimulus auditif et les notations de l'utilisateur. Les moyens de synchronisation 4 comprennent ici un ordinateur 5, sur lequel est installé un logiciel :
- assurant la synchronisation du stimulus sonore, correspondant à fichier sonore, à évaluer et du signal d'évaluation subjective continue issu du dispositif de notation en temps réel 3 ;
- acceptant en entrée un fichier de stimulus (par exemple des fichiers .wav pour des enregistrements sonores) ;
- permettant une sélection partielle du stimulus pour en n'évaluer qu'une partie,
- fournissant en sortie un fichier contenant l'évaluation subjective projetée sur l'échelle de cotation choisie en fonction du temps.

Pour assurer la synchronisation du fichier sonore à évaluer et le signal d'évaluation, l'ordinateur 5 peut être agencé :
- pour commander la diffusion ou l'émission du stimulus auditif, via le haut parleur 1a ; et
- pour recevoir les notations en provenance des moyens de notation 3.

Le dispositif de notation en temps réel 3 va maintenant être décrit plus précisément en référence aux figures 2 et 3 qui illustrent un mode de réalisation de ce dispositif.

Le dispositif de notation en temps réel 3 comprend un bouton 31 monté rotatif sur un boîtier 33. Le boîtier 33 présente une échelle graduée 32 associée à la rotation du bouton 31. L'échelle graduée 32 permet à l'utilisateur de repérer visuellement le déplacement du bouton 31 et, par suite, la note qu'il attribue. Le boîtier 33 est adapté pour tenir dans une main.

Le bouton 31 et le boîtier 33 présentent des crans aptes à coopérer en cas de rotation du bouton 31 par rapport au boîtier 33. La distance entre ces crans correspond de préférence à une division d'une graduation de l'échelle graduée 32. Ces crans permettent à l'utilisateur du dispositif de notation d'évaluer plus finement le stimulus auditif auquel il est soumis. Ces crans permettent en effet à l'utilisateur d'intégrer, via le passage des crans, un pouvoir discriminant fin, capable de distinguer de manière sensible toute discontinuité du ressenti au cours du stimulus à évaluer.

Le boîtier 33 est muni d'un codeur (non représenté) du déplacement du bouton 31. Le déplacement du bouton 31 peut ainsi être codé, par exemple en un signal électrique, pour être ensuite transmis à une unité de traitement (en l'espèce l'ordinateur 5) par des moyens de transmission 35 permettant l'envoi de l'information codée vers l'unité de traitement.

Le boîtier 33 comporte encore un dispositif de retour d'effort agissant sur le bouton 31. Ce dispositif de retour d'effort est de préférence du type variable, qui exerce un effort sur le bouton qui est fonction du déplacement du bouton 31 par rapport au boîtier 33. L'effort peut notamment être proportionnel au déplacement du bouton 31 par rapport au boîtier 33. Ainsi, ce dispositif de retour d'effort permet d'obtenir une information mécanique sur la position du bouton rotatif. Ce dispositif de retour d'effort oblige en effet l'utilisateur à réaliser un effort plus important pour un même déplacement angulaire (c'est-à-dire une même variation de note) aux extrema de l'échelle graduée qu'au voisinage de la position neutre du bouton.

Le dispositif de notation en temps réel 3 comporte encore des moyens de retour du bouton 31 dans une position neutre. Ces moyens de retour, qui peuvent également avoir la fonction de dispositif de retour de force, peuvent par exemple prendre la forme de deux ressorts de rappels montés entre le boîtier 33 et le bouton 31 de telle sorte que, dès que le bouton 31 est déplacé par rapport à sa position neutre, l'un des ressort agisse sur le bouton 31 par traction alors que l'autre ressort agit sur le même bouton 31 pas repoussement. En d'autres termes, dès que le bouton est déplacé de sa position neutre, l'un des ressorts est comprimé, l'autre étant étiré.

En variante, les moyens de retour, qui ont également la fonction de dispositif de retour de force, comportent deux ressorts de rappels agissant de manière séquentielle selon le sens de rotation du bouton 31 par rapport au boîtier. En d'autres termes, un seul ressort agit à la fois sur le bouton 31, en fonction du sens de rotation du bouton par rapport à sa position neutre. L'action de ces ressorts se traduit par une force de rappelle opposée au mouvement de rotation du bouton 31 par rapport au boîtier 33. Cette action peut être proportionnelle à l'angle de rotation du bouton 31 par rapport à sa position neutre. Le couple que l'utilisateur doit appliquer pour faire tourner le bouton est alors plus important au fur et à mesure que l'évaluation approche des notes extrêmes de l'échelle graduée. Cette perception d'effort permet de fiabiliser l'évaluation subjective de l'utilisateur dans les extrema. En effet, pour atteindre ces extrema, l'utilisateur doit vaincre un effort plus important pour confirmer de manière consciente sa volonté de donner une évaluation extrême (c'est-à-dire très positive ou au contraire très négative) en traduction de son ressenti au stimulus.

Le dispositif de notation en temps réel 3 permet ainsi à son utilisateur d'associer à un bruit perçu à un instant donné, une note de l'échelle graduée en déplaçant le bouton 31 simultanément à la perception du bruit. La notation est immédiatement, c'est-à-dire en temps réel, transformée en signal électrique et émise vers l'ordinateur 5 via les moyens de transmissions 35.

Le système de notation en temps réel peut être mis en oeuvre de la manière suivante. Le dispositif de notation en temps réel 3 est mis à disposition de l'utilisateur 2 soit sur une console fixe, soit dans la main de l'utilisateur 2. Le haut parleur 1a, commandé par l'ordinateur 5, émet le stimulus auditif vers l'utilisateur. Ce dernier note, en temps réel, chaque bruit entendu grâce au dispositif de notation 3.

En parallèle, l'ordinateur 5 enregistre les notes de l'utilisateur 2 et effectue une synchronisation du stimulus auditif et de la notation dudit stimulus auditif de sorte à obtenir une corrélation entre au moins une portion du stimulus auditif et une notation associée.

Il en résulte un diagramme où chaque bruit du stimulus est associé à une note. Le procédé, répété avec plusieurs utilisateurs, permet d'obtenir un profil de bruit noté dans lequel chaque bruit est associé à une valeur reflétant le plaisir ou la gêne occasionnée par le bruit ainsi qu'une moyenne de l'intensité ressentie.

La présente invention n'est pas limitée aux exemples et au mode de réalisation décrits et/ou représentés, mais elle est susceptible de nombreuses variantes accessibles à l'homme de l'art.

Le système de notation est particulièrement adapté à un environnement automobile car il permet d'alimenter en données subjectives (données utilisateur) des logiciels de simulation auditifs. Toutefois, ce dispositif peut être utilisé dans tous les domaines de la psycho acoustique.

En outre, le dispositif et le système de notation en temps réel peuvent être mis en oeuvre pour évaluer tout type de stimuli.

Enfin, le dispositif de notation en temps réel décrit comprend un bouton monté rotatif, ce qui permet d'obtenir un dispositif particulièrement compact. Cependant, le bouton peut être apte à se déplacer selon d'autres mouvements qu'une rotation par rapport au boîtier, notamment en translation.

## Revendications

1. Dispositif (3) de notation en temps réel d'un stimulus, **caractérisé en ce qu'il** comporte un bouton (31) apte à être déplacé par rapport à un boîtier (33), le boîtier (33) présentant une échelle graduée (32) associée au déplacement du bouton (31).

2. Dispositif de notation selon la revendication 1, dans lequel le bouton (31) et le boîtier (33) présentent des crans aptes à coopérer en cas de rotation du bouton (31) par rapport au boitier (33), la distance entre deux crans correspondant à une division d'une graduation de l'échelle graduée.

3. Dispositif de notation selon la revendication 1 ou 2, comprenant un dispositif de retour d'effort agissant sur le bouton (31).

4. Dispositif de notation selon la revendication 4, dans lequel le dispositif de retour d'effort est du type variable, le retour d'effort étant fonction du, de préférence proportionnel au, déplacement du bouton (31).

5. Dispositif de notation selon l'une quelconque des revendications précédentes, comprenant des moyens de retour du bouton (31) en une position neutre du bouton (31).

6. Dispositif de notation selon les revendications 5 et 6, dans lequel le dispositif de retour d'effort et les moyens de retour du bouton en une position neutre du bouton sont formés de deux ressorts de rappels montés entre le boîtier (33) et le bouton (31).

7. Dispositif de notation selon l'une quelconque des revendications précédentes, comprenant des moyens de codage du déplacement du bouton et des moyens de transmission (35) du déplacement codé vers une unité de traitement.

8. Dispositif de notation selon l'une quelconque des revendications précédentes, dans lequel le bouton (31) est monté rotatif sur le boîtier (33).

9. Système de notation en temps réel d'un stimulus, notamment d'un stimulus auditif, de préférence encore d'un bruit de combustion d'un moteur Diesel d'un véhicule automobile, comprenant :
- des moyens d'émission (1) vers un utilisateur (2) du stimulus à évaluer ;
- un dispositif de notation (3) selon l'une quelconque des revendications précédentes ; et
- des moyens de synchronisation (4) pour synchroniser le stimulus et les notations de l'utilisateur.

10. Système de notation d'un stimulus selon la revendication 9, dans lequel les moyens de synchronisation (4) comportent un ordinateur (5) agencé pour :
- commander la diffusion du stimulus ; et
- recevoir les notations de l'utilisateur en temps réel.

11. Procédé de notation en temps réel d'un stimulus, notamment d'un stimulus auditif, de préférence d'un bruit de combustion d'un moteur Diesel d'un véhicule automobile, mis en oeuvre au moyen d'un système de notation selon la revendication 9 ou 10, comprenant les étapes de :
- émission par les moyens d'émission d'un stimulus vers un utilisateur (2) ;
- notation par l'utilisateur (2) en temps réel du stimulus ;
- synchronisation du stimulus et de la notation du stimulus de sorte à obtenir une corrélation entre au moins une portion du stimulus et une notation associée.
